# EUROPEAN PATENT APPLICATION

(11) **EP 2 163 900 A1**
(43) Date of publication of application: **17.03.2010**
(21) Application number: 08305526.9
(22) Date of filing: 04.09.2008
(51) Int. Cl.: G01N 33/68, G01N 33/92, G01N 33/532, C09B 11/04

(54) **New method of imaging by mass spectrometry and new mass tag associated trityl derivatives**

(71) Applicant: COMMISSARIAT A L'ENERGIE ATOMIQUE, 75015 Paris (FR)
(72) Inventor: Gut, Ivo Glynne, 75014 Paris (FR)
(74) Representative: Warcoin, Jacques

(57) **Abstract**

The present invention concerns a method of analyzing at least one specific molecule in a sample using a compound of formula (I) 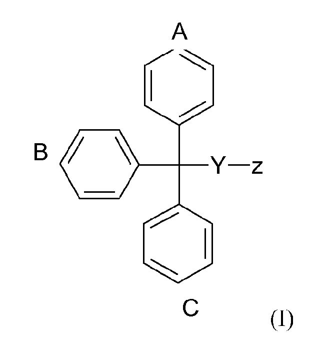 wherein Z binds specifically to said at least one specific molecule, Y is independently a cleavable single bond, linker atom or group, and at least one of the cycles A, B or C is substituted; and such a compound (I).

## Description

### Field of the Invention

The present invention relates generally to the fields of medical imaging, analysis, monitoring and diagnostics. More specifically, it provides methods for analyzing proteins in samples and also new trityl-type compounds and their use as mass tag for solution and solid support applications.

### Background of the invention

Immunohistochemical methods were first shown in 1942 in the work of COONS et al. (J. Immunol., vol.45, p:159-170, 1942). They presented a method employing the specificity of an antibody labelled with fluorescein for the localization of antigens under a fluorescence microscope. It was a generic method for the histological localization of any antigen of interest. The antibody molecule could be conjugated with simple chemical compounds without destroying its capacity to react specifically with its antigen. Tissue sections were incubated with dilute, specific antibody solutions. Any antigen present bound the antibody and fixed it in place. Excess reagent was washed off and the histochemical reagent could be localized by irradiation with light of appropriate wavelength and visualized. This principle has been extended for use with an electron microscope by antibodies labelled with ferritin or with enzymes such a horseradish peroxidase. Immunohistochemistry (IHC) has become progressively more applied in many biological and medical areas. Nowadays, immunohistochemical methods are widely used for routine diagnostics in pathology (CERIO et al., J. Invest. Dermatol., vol.87, p:499-503, 1986; CERIO & MACDONALD, J. Clin. Lab. Immunol., vol.20, p:97-100, 1986; CERIO & MACDONALD, Adv. Dermatol., vol.3, p:123-140, 1988; CERIO & WILSON-JONES, Clin. Exp. Dermatol., vol. 14, p:177-180, 1989).

Ten years ago a new concept for imaging tissue sections was bomimaging MS (IMS). PRIOLI *et al.,* (CAPRIOLI et al., Anal. Chem., vol.69, p :4751-4760, 1997; CHAURAND & CAPRIOLI, Electrophoresis, vol.23, p :3125-3135, 2002; CHAURAND et al., Am. J. Pathol., vol.165, p :1057-1068, 2004 ; TODD et al., J. Mass Spectrom., vol.36, p :355-369, 2001) used MALDI-TOF MS to generate the first mass spectrometric images of tissue sections. Mass spectrometers were adapted for scanning and many further required elements, such as methods for depositing matrix (SUGIURA et al., Anal. Chem., vol.78, p :8227-8235, 2006 ; AERNI et al., Anal. Chem., vol.78, p:827-834, 2006 ; HANKIN et al, J. Am. Soc. Mass Spectrom., vol.18, p:1646-1652, 2007), procedures for increasing spatial resolution (CHAURAND et al, J. Mass Spectrom., vol.42, p:476-489, 2007 ; KLINKERT et al., Rev. Sci. Instrum., vol.78, p:053716, 2007; JURCHEN et al., J. Am. Soc. Mass Spectrom., vol.16, 1654-1659, 2005; ALTELAAR et al., Int. J. Mass Spectrom., vol.206, 203-211, 2007), the SMALDI, by SPENGLER & HUBERT (J. Am. Soc. Mass Spectrom., vol.13, 735-748, 2002) which is capable of decreasing the irradiated area to a diameter ,500 nm, and software (CLERENS et al., Rapid Commun. Mass Spectrom., vol.20, 3061-3066, 2006) to assemble images at any mass of interest were developed. For a recent comprehensive review see Cornett et al. (CORNETT et al., Nat. Methods, vol.4, 828-833, 2007). Later, other laboratories (ALTELAAR et al., Anal. Chem., vol.77, 735-741, 2005 ; MCDONNELL et al., J. Mass Spectrom., vol.40, 160-168, 2005 ; TOUBOUL et al., J. Am. Soc. Mass Spectrom., vol.16, 1608-1618, 2005) started experimenting with other forms of desorption, such as secondary ion MS (SIMS) (BENNINGHOVEN & SICHTERMANN, Anal. Chem., vol.50, 1180-1184, 1978).

The concept of TArgeted multiplex MS IMaging (TAMSIM; THIERY et al., Rapid Commun. Mass Spectrom., vol.21, p:823-829, 2007) is the combination of IHC and IMS to provide a method for imaging multiple candidate antigens simultaneously. Thus, for TAMSIM, an antibody molecule is conjugated with a photocleavable mass tag employed as histochemical revealing reagent. Tags are released from their respective antibodies by a laser pulse at 355 nm without having added matrix. After scanning MS images are created for the mass of each tag. Recently other demonstrations of this approach were presented (LEMAIRE et al., J. Proteome Res., vol.6, p :2057-2067, 2007; WISZTORSKI et al., Med. Sci., vol.23, p:31-36, 2007 ; YANG et al., Proceedings of the 55th ASMS Conference on Mass Spectrometry and Allied Topics, 2007).

Nevertheless, there is still a need for new method enabling the detection of several markers on the same section, so as to be able to compare this with individual positive control for a diagnosis or prognosis purpose for example.

### Summary of the invention

The present invention relates to a method of analyzing at least one specific molecule in a sample comprising the step of:
a) providing a sample;
b) contacting said sample with at least one compound of formula (I) 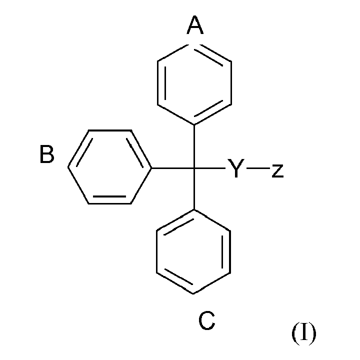 wherein Z binds specifically to said at least one specific molecule, Y is independently a cleavable single bond, linker atom or group, and at least one of the cycles A, B or C is substituted;
c) exposing the sample to a laser beam such that a predetermined laser spot on the sample induces the cleavage of Y to form and released an Ion of formula (II): 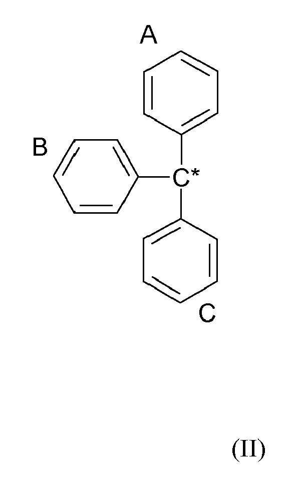 wherein * corresponds to a single positive charge or a single negative charge bearded by the carbon atom , preferably a positive charge;
d) measuring the molecular atomic mass of the released compounds over a range of atomic mass so as to identify and, eventually, quantify the Ion of formula (II);
e) repeating the steps c) to d) to set up an effective scan of the sample; and
f) determining the spatial arrangement and, eventually, the quantity of the at least one specific molecule within the sample.

The present invention further relates to a compound of formula (I): 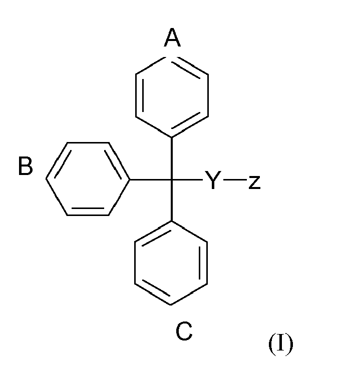 wherein Z binds specifically to the at least one specific molecule as disclosed previously, Y is independently a cleavable single bond, linker atom or group, and at least one of the cycles A, B or C is substituted.

### Brief Description of the Drawings

Figure 1 shows the Concept of TAMSIM.

Figure 2 shows the Conjugation of a mass tag to an antibody, photocleavage of mass tag conjugated-antibody, and laser desorption.

Figure 3 shows the Imaging of cells immunoreactive with polyclonal antisynaptophysin antibody in healthy human pancreas.

Figure 4 shows Imaging of cells immunoreactive with polyclonal rabbit anti-human chromogranin A antibody and monoclonal mouse anti-human insulin in Langerhans islets in a human pancreas paraffin embedded tissue section.

Figure 5 shows Imaging of cells immunoreactive with polyclonal rabbit anti-human chromogranin A antibody and monoclonal mouse anti-human insulin in Langerhans islets in a frozen section of human pancreas.

Figure 6 shows Imaging of cells immunoreactive with monoclonal rabbit anti-human calcitonin antibody, monoclonal rabbit anti-human synaptophysin and polyclonal rabbit anti-human somatostatin antibody in Langerhans islets in a frozen section of human pancreas.

Figure 7 shows the determination of the tag E1 307 sensitivity threshold.

### Detailed Description

The inventors have now developed new improvements for TAMSIM enabling multiplex imaging of target proteins in histological sections.

The first is the use of direct IHC. In contrast to TAMSIM, where the mass tags were added to secondary antibodies, the primary antibody is directly conjugated with the histochemical reagent and incubated with the tissue section. Direct IHC has an advantage for multiplexing as it is not limited by the number of species available for antibody production.

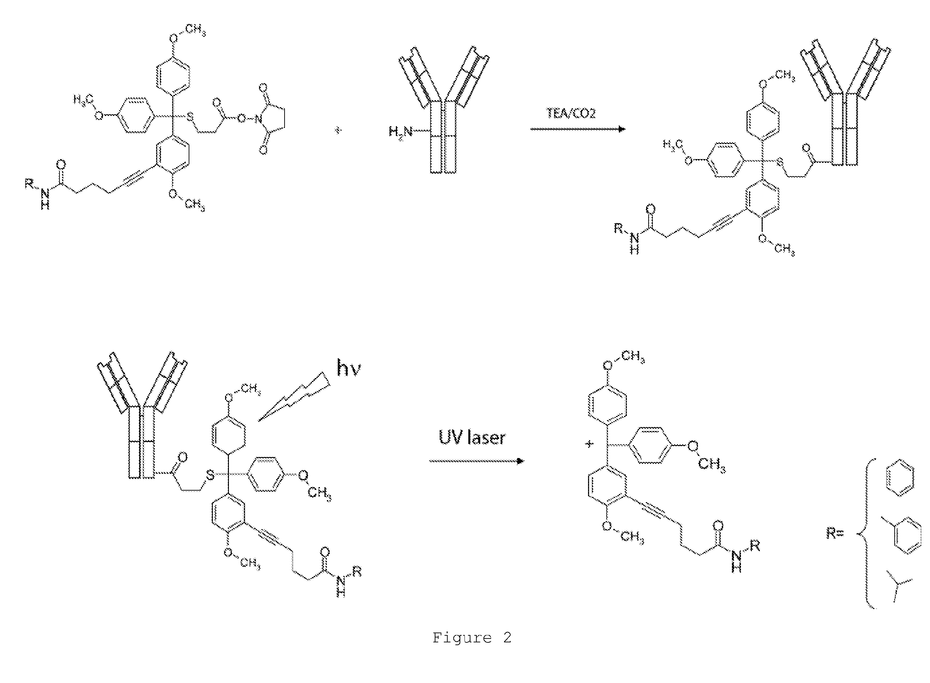

The second improvement is the preparation of a second generation of photocleavable tags. This new class of tags having alkyl or aromatic groups for mass tuning (residue R in Fig. 2) is different than the previously used tags at the level of the amide group. This structure leads to the stabilization of R on the tag, said tags being more stable which facilitates handling. In fact, fewer fragments were observed in gas phase with these new tags. More generally, these tags bring several advantages over, for example, the tags developed by OLEJNIK *et al.* (OLEJNIK et al., Nucleic Acids Res., vol.24, p:361-366, 1996; OLEJNIK et al., Nucleic Acids Res., Vol.27, p:4626-4631, 1999) such as (i) cleavage takes place during the laserpulse, (ii) the tag structure with its functionalized trityl groups acts as "matrix" and as a result no matrix deposition is necessary, (iii) the tags are very easy to detect as a carbocation is created during laser desorption which is amenable to facile TOF analysis.

The third improvement is the application of fresh frozen sections, which reduces artefact peaks in the mass spectra in contrast to paraffin-embedded sections.

The last improvement is an enhancement of the degree of multiplexing: three markers were able imaged in the same tissue section.

Consequently, in one aspect the present invention relates to a method of analyzing at least one specific molecule in a sample comprising the step of:
a) providing a sample;
b) contacting said sample with at least one compound of formula (I) 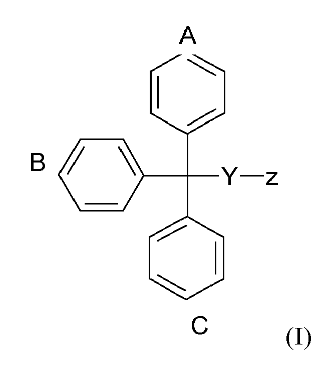 wherein Z binds specifically to said at least one specific molecule, Y is independently a cleavable single bond, linker atom or group, and at least one of the cycles A, B or C is substituted;
c) exposing the sample to a laser beam such that a predetermined laser spot on the sample induces the cleavage of Y to form and released an Ion of formula (II): 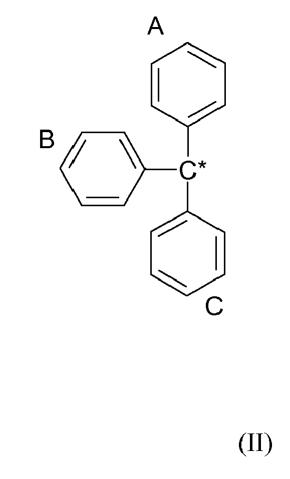 wherein * corresponds to a single positive charge or a single negative charge bearded by the carbon atom , preferably a positive charge;
d) measuring the molecular atomic mass of the released compounds over a range of atomic mass so as to identify and, eventually, quantify the Ion of formula (II);
e) repeating the steps c) to d) to set up an effective scan of the sample; and
f) determining the spatial arrangement and, eventually, the quantity of the at least one specific molecule within the sample.

As used herein, the term specific molecule corresponds to specific nucleic acid, lipid, carbohydrate, peptide or polypeptide. Preferably, said specific molecule is a specific antigen selected in the group consisting of lipids, carbohydrates, peptides and polypeptides. More preferably, said specific molecule is a peptide or a polypeptide, such as synaptophysin, chromogranin, insulin, calcitonin or somatostatin.

In one embodiment, the sample may be a tissue section from a specific tissue of interest. The sample may also be individual cells or clusters, which may be isolated by laser-capture microdissection. Some examples of tissues that may be analyzed include, but are not limited to, testicular, prostate, lung, breast, colon, and brain cancer. The tissue may be animal or human tissue, and it may be normal or tumor-bearing tissue. Tissue sections may be obtained by any means known in the art, including surgical means. If a tissue is obtained surgically, it is advantageous that the tissue be intact and the location of the tissue be known prior to removal. As an example, and if the tissue is a tumor-bearing tissue, the techniques described herein may be used in intra-operative assessment of the surgical margins of tumors. Tissue may also be obtained from tissue grown in any medium, and the tissue obtained may be stored for later analysis for an indefinite period of time according to methods known in the art. With the benefit of this disclosure, those having skill in the art will recognize that other types of specimens and tissue may be analyzed using the very techniques described herein, without insubstantial modifications.

The tissue section may be paraffin-embedded tissue section or frozen tissue section (i.e., not paraffin-embedded tissue section), and preferably said tissue section is a frozen tissue section. Preferably, said tissue section is less than 50 µm, and more preferably said tissue section is 5 µm to 16 µm thick.

The sample may or may not include an energy absorbent matrix, which is a material that will absorb UV or energy at other wavelengths. This energy absorbent matrix may include an organic or inorganic compound having a relatively high extinction coefficient for absorption of energy and may be applied in a thin layer over the sample or otherwise be incorporated in the sample. Example energy absorbent matrixes include but are not limited to 2,5-dihdroxybenzoic acid and alpha-cyano-4-hydroxycinnamic acid. The energy absorbent matrix may be applied using electrospray, pneumatic spray, spin coating, dip coating or any other appropriate method.

The compound of formula (I) corresponds to a trityl derivative showing enhanced ionisability, which ionization results in the formation of the ion of formula (II) allowing analysis by mass spectroscopy. Because of the enhanced ionisability of compound of formula (I), an additional energy absorbent matrix may not be required. Thus, ionization may be obtained without requiring acid treatment.

In one embodiment, the method may not include the step of applying an additional energy absorbent matrix to the sample. Here, the energy is absorbed only by the sample and is not first incident on an exogenous energy absorbing matrix. Thus, sample preparation is simpler than prior art method in that the additional step of applying the matrix is not required.

The nature of Z in formula (I) depends on the nature of the specific molecule to which it binds. As an example, if the specific molecule is a specific nucleic acid, then Z can be a complementary acid nucleic, if the specific molecule is a specific antigen, then Z can be an antibody or a functional fragment thereof which binds specifically to this specific antigen.

The synthesis of compound of formula (I) can be done by the skilled person according to well known methods. Such methods are disclosed as an example in International patent application PCT WO 01/72926, WO 2005/057207 or WO 2006/032893, or in SHCHEPINOV et al., (Nucleic Acids Symp. Ser., vol.42, p:107-108, 1999).

Z, before its linkage to Y in compound of formula (I), has at least one reactive group so as to form a covalent linkage for obtaining a compound of formula (I). Such groups typically include naturally occurring groups and groups formed synthetically on Z.

Naturally occurring groups and groups formed synthetically are well known from the skilled person and include, as an example, those presented in International patent application PCT WO 2006/032893 (page 30, line 25 to page 33, line 27) which is incorporated herein by reference.

Y, before its linkage to Z in compound of formula (I), comprises a reactive functional group.

Such reactive functional groups are well known from the skilled person and include, as an example, those presented in International patent application PCT WO 2006/032893 (page 34, line 14 to page 37, line 21) which is incorporated herein by reference.

In one embodiment, Z is an antibody or a functional fragment thereof.

The term "functional fragments" as used herein refers to antibody fragment capable of binding specifically with the antigen. Such fragments can be simply identified by the skilled person and comprise, as an example, F_{ab} fragment (e.g., by papain digestion), F_{ab}' fragment (e.g., by pepsin digestion and partial reduction), F(_{ab}')₂ fragment (e.g., by pepsin digestion), F_{acb} (e.g., by plasmin digestion), F_{d} (e.g., by pepsin digestion, partial reduction and reaggregation), and also scFᵥ (single chain Fv; e.g., by molecular biology techniques) fragment are encompassed by the invention.

Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a F(_{ab}')₂ heavy chain portion can be designed to include DNA sequences encoding the CH₁ domain and/or hinge region of the heavy chain. The various portions of antibodies can be joined together chemically by conventional techniques, or can be prepared as a contiguous protein using genetic engineering techniques.

The expression "binding specifically to the molecule" and "capable of binding specifically to the antigen" refers to a K_{D} of less than 10⁻⁶M, preferably from less than 10⁻⁸ M, and more preferably less than 10⁻¹⁰M for this molecule or antigen.

Y is cleavable by irradiation, electron bombardment, electrospray, fast atom bombardment (FAB), inductively coupled plasma (ICP) or chemical ionization. Preferably Y is cleavable by irradiation or chemical ionization.

Y does not comprise any aromatic group.

Preferably, Y is independently -Y¹-, -C(=Y¹)-, -Y¹C(=Y¹)-, -C(=Y¹) Y¹-, - Y¹C(=Y¹)Y¹-, -S(=O)-, -Y¹S(=O)-, -S(=O)Y¹-, -Y¹S(=O)Y¹-, -S(=O)₂-, -Y¹S(=O)₂-, - S(=O)₂Y¹-, -Y¹S(=O)₂Y¹, where Y¹ is independently O, S or N(R¹), and where R¹ is independently H, C₁₋₈ hydrocarbyl {e.g. C₁₋₈ alkyl) or C₁₋₈ hydrocarbyl substituted with one or more D.

More preferably, Y is independently -O-, -S-, -C(=O)-, -C(=O)O-, -C(=S)-, - C(=S)O-, -OC(=S)- -C(O)S-, -SC(=O)-, -S(O)-, -S(O)₂-, -N(R₁)-, -C(=O)N(R₁)-, - C(=S)N(R₁)-, -N(R₁)C(=O)-, -N(R₁)C(=S)-, -S(=O)N(R₁)-, -N(R₁)S(=O)-, - S(=O)₂N(R₁)-, -N(R₁)S(=O)2-, -OC(=O)O-, -SC(=O)O-, -OC(=O)S-, -N(R₁)C(=O)O-, - OC(=O)N(R₁)-, -N(R₁)C(=O)N(R₁)-, -N(R₁)C(=S)N(R₁)-, -N(R₁)S(=O)N(R₁)-, - S(R₁)C(=O)N- or -N(R₁)S(=O)2N(R₁)-.

In one embodiment, the method comprise the step b) of contacting said sample with a compound of formula (I ')

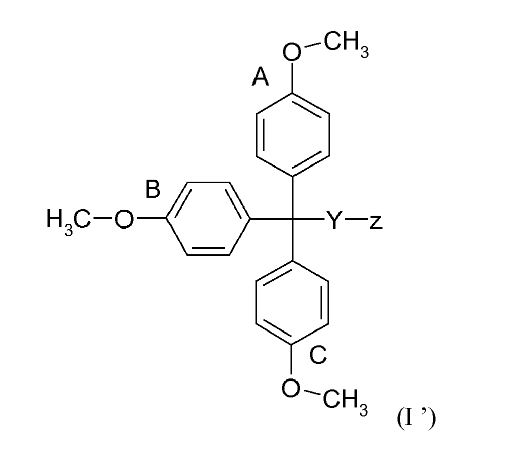

Which compound of formula (I '), after exposing of the sample to a laser beam so as to induce the cleavage of Y in step d), form a released Ion of formula (II'):

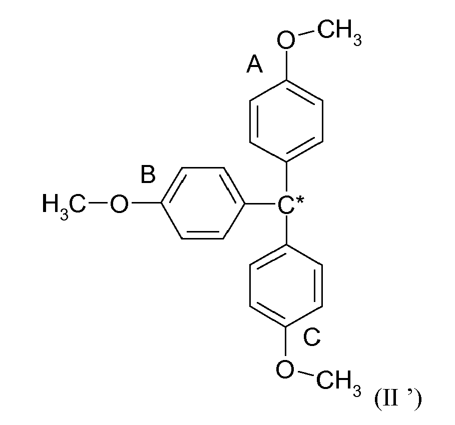

In one other embodiment, the method comprise the step b) of contacting said sample with a compound of formula (I "):

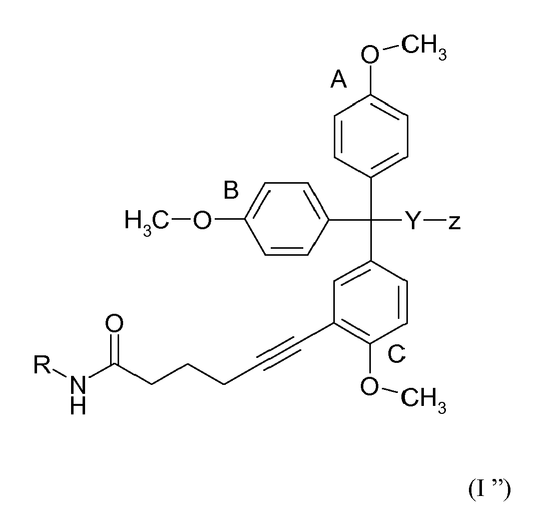

Which compound of formula (I "), after exposing of the sample to a laser beam so as to induce the cleavage of Y in step d), form a released Ion of formula (II"): 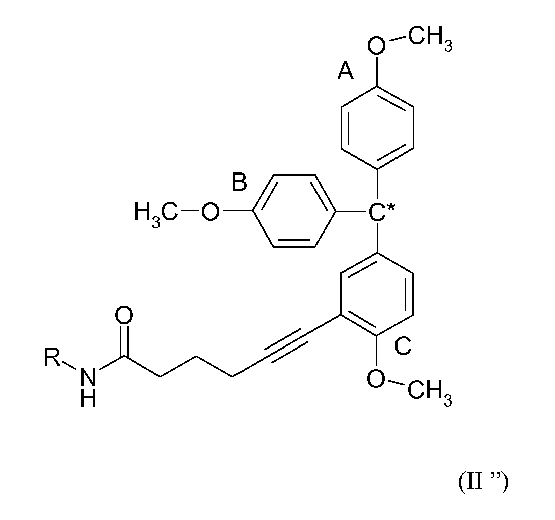 wherein R is independently a substituent such as H, C₁₋₂₀ hydrocarbyl {e.g. C₁₋₂₀ alkyl, C₁₋₂₀ aryl) or substituted C₁₋₂₀ hydrocarbyl. Preferably, R is H, C₁₋₂₀ alkyl, C₁₋₂₀ aryl, substituted C₁₋₂₀ alkyl or substituted C₁₋₂₀ aryl. More preferably, R is an isopropyl or a phenyl substituted or not with a methyl.

Typically, the steps c) to e) is carried out with mass spectroscopy in a spectrometer.

In step c), the sample is exposed to a laser beam laser. Prior to this step, the sample may be dried. The laser is configured so that it strikes a predetermined spot on the sample, which releases Ion of formula (II) from the sample. The size and position of the laser spot may be varied as it is known in the art. A laser mask may also be used for selectively shaping or defining the size of laser spots on a test sample. Such a mask may block parts of the laser beam not intended for use so that the beam profile is well defined in both shape and size when it is incident on the sample. As an example of such a mask, one can cite the one disclosed in International patent application PCT WO 2007/128751. The type of laser and its power settings may likewise be adjusted as is known in the art.

In the spectrometer, the ion source may be a matrix-assisted laser desorption ionization (MALDI), an electrospray ionization (ESI) ion source, a Fast-atom bombardment (FAB) ion source. Preferably, the ion source is a MALDI ion source.

The MALDI ion source may be traditional MALDI source (under vaccum) or may be an atmospheric pressure MALDI (AP-MALDI) source.

In the spectrometer, the mass analyzer may be a time of flight (TOF), quadruopole time of flight (Q_TOF), ion trap (IT), quadruopole ion trap (Q-IT), triple quadruopole (QQQ) Ion Trap or Time-Of-Flight Time-Of-Flight (TOFTOF) or Fourrier transform ion cyclotron resonance (FTICR) mass analyzer.

Preferably, the mass spectrometer is a MALDI-TOF mass spectrometer.

In step e), the sample is moved relative to the laser beam. This translation may be by a predetermined distance. This distance may be functionally related to the size of the laser spot to achieve an effective scan pattern. By varying the amount of the translation, one may affect the resolution of the scan, as is known in the art. The mechanism used to translate the sample may be any one of a number of translation stages available commercially. The type of translation may be one, two, or three dimensional, depending on the application. In one embodiment, the distance of movement between successive laser spots may be less than twice the width of each of the successive laser spots. As an example of disclosure of such step e), one can cite, as an example, the "translation" step of the methods disclosed in patents US 5,808,300 and US 6,756,586.

The step f) of assessing the spatial arrangement and, eventually, the quantity of the at least one specific molecule on the sample is done by inputting the atomic mass data for the Ion of formula (II) obtained for each laser spot during steps c) to e) to a computer, and the atomic mass of the compound of formula (II), (II ') or (II ") is then depicted as a function of individual laser spots on the test sample. This step f) enables to generate an X,Y two dimensional pattern of the at least one specific molecule corresponding to the X,Y two dimensional pattern of the Ion of formula (II) on the sample and successive sample sections can be analyzed to generate an X,Y,Z three dimensional pattern.

As will be understood by those having skill in the art, data analysis steps may be undertaken while additional scans are being made. In other words, data processing may take place at the same time as the sample is being scanned.

In other embodiments, the method of the invention is a method of analyzing at least two, three, four or more specific molecules in a sample comprising the steps disclosed previously, with a step b) of contacting said sample with at least two, three, four or more compounds of formula (I), each compound of formula (I) binding specifically to each specific molecule to be analyzed.

The method of the invention has vast applications in the imaging, monitoring, diagnosis, and treatment of a myriad of disorders. In some embodiments, specific tumor markers may be analyzed, imaged, identified, and monitored for diagnostic and/or treatment regimes.

In a second aspect, the present invention relates to a compound of formula (I) 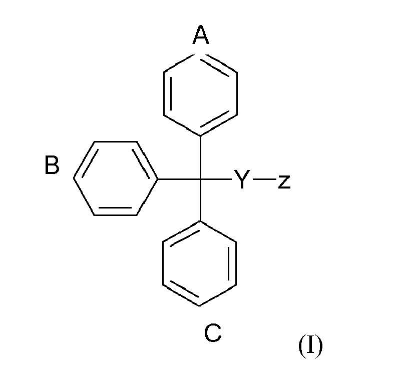 wherein Z binds specifically to the at least one specific molecule as disclosed previously, Y is independently a cleavable single bond, linker atom or group, and at least one of the cycles A, B or C is substituted.

Said compound of formula (I) is as disclosed previously.

In one embodiment, said compound has the formula (I '):

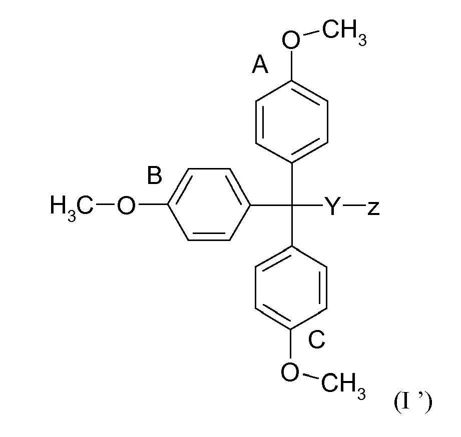

In another embodiment, said compound has the formula (I "):

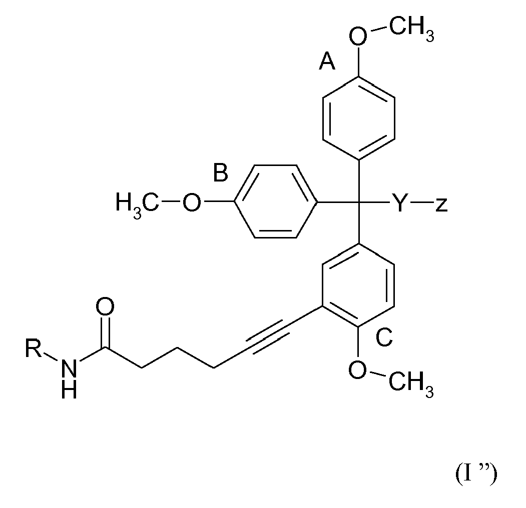

Said compound of formula (I ") is as disclosed previously.

Unless defined otherwise, all technical and scientific terms used herein have the same meaning as is commonly understood by one of the skill in the art to which this invention belongs.

The present invention may be better understood by reference to the following non- limiting Examples, which are provided as exemplary of the invention. The following examples are presented in order to more fully illustrate the preferred embodiments of the invention. They should in no way be construed, however, as limiting the broad scope of the invention.

### EXAMPLES

### 1) Materials & methods:

### 1.1 Tag synthesis

The synthesis of the tags, 2,5-dioxopyrrolidin-1-yl-3-{[3-(6-(tert-butylamino)-6-oxo hex-1-ynyl)-4-methoxyphenyl)bis(4-methoxyphenyl)]methylthio}propanoate here called El 307, 2,5-dioxopyrrolidin-l-yl-3-{[3-(6-(benzylamino)-6-oxohex-l-ynyl)-4-methoxyphenyl) bis(4-methoxyphenyl)]methylthio} propanoate here called El 308, and 2,5-dioxopyrrolidin-1-yl-3-{[3-(6-(phenylamino)-6-oxohex-1-ynyl) -4-methoxyphenyl)bis(4-ethoxyphenyl)] methylthio}propanoate here called JC14-110, were carried out in analogy to the method described by SHCHEPINOV et al., (Nucleic Acids Symp. Ser., vol.42, p:107-108, 1999).

### 1.2 Preparation of tissue sections

Routine pancreas biopsies from patients were divided. One part was embedded in paraffin and the other frozen. Frozen sections and tissue embedded in paraffin were cut (4 mm) and mounted on slides (Service d0Anatomie Pathologique, H_pital Cochin, Paris, France). For use paraffin-embedded sections were deparaffinized with xylene and frozen sections thawed. Antibodies used here were commercially available rabbit monoclonal antisynaptophysin, monoclonal mouse anti-insulin, monoclonal rabbit anticalcitonin, polyclonal rabbit antisomatostatin (Microm Microtech, Francheville, France) and polyclonal rabbit antihuman chromogranin A (DAKO, Trappes, France). Antibodies were diluted between 1:50 and 1:400 with 1X PBS.

### 1.3 Immunoenzyme control staining

After deparaffinisation sections were incubated with 10 mM citrate buffer to unmask antigen epitopes. Endogenous peroxidise activity was blocked with 3% H2O2. Sections were incubated with rabbit monoclonal antisynaptophysin diluted 1:50. After 30 min of incubation at room temperature with the primary antibody, the binding was visualized using biotinylated swine antirabbit immunoglobulin diluted 1:100 (DAKO). Horseradish peroxidase-labeled streptavidin was attached to the biotin-labeled antibody (DAKO). The complex was visualized by the enzymatic reaction of peroxidise and the chromogen DAB (30-diaminobenzidine tetrahydrochloride).

Frozen sections were thawed at room temperature and treated with acetone. Aceton is used to immobilize antigens on the tissue section. Sections were incubated with antiinsulin (polyclonal guinea pig antiswine insulin) antibody diluted 1:100. After 30 min of incubation at room temperature with the primary antibody, binding was visualized using a biotinylated secondary antibody phosphataselabeled streptavidin-biotin complex and Fast Red was used as a chromogen. The staining was visualized by light microscope.

### 1.4 Immunohistochemistry with photocleavable tags

The reaction can be scaled for any amount of protein, but the concentration of the protein should be at least 2 mg/mL for optimal results. The antibody was diluted 1:50 in PBS (pH = 7.6). The pH was adjusted between 8 and 9 by the addition of triethylamine/CO2. The tag N-hydroxysuccinimide (NHS) esters were dissolved in anhydrous dimethylsulfoxide at a concentration of 10 mM. Three different tags were used: El 308 Mw = 734 Da, corresponding mass tag = 532 Da; El 307 Mw = 700 Da, corresponding mass tag = 498 Da; JC14-110 Mw = 720 Da, corresponding mass tag = 518 Da. Between 5 and 10 mL of tag NHS ester was added to the antibody solution (50-100 mL) at pH = 8-9 and the reaction mixture was incubated for 2 h on ice. Nonreacted tag was removed with Micro Bio-Spin Chromatography Columns (BIORAD) according to manufacturers' instructions. After incubation at room temperature for between 30 min and 1 h with primary antibodies conjugated with mass tags, slides were washed with PBS for 5-10 min and rinsed once with distilled water. Finally, the slides were mounted directly onto a metal MALDI target plate and introduced into the source of the mass spectrometer. In order to avoid an offset between the two images, IHC control and TAMSIM were carried out on the same tissue sections.

### 1.5 Determination of tag sensitivity

A dilution series of the tag El 307 was prepared (starting from a 566 mM solution of the tag, ten-fold dilutions to 0.056 mM and from there on three-fold dilutions to 7.861022 nM). The solutions were deposited on three pancreas tissue sections (nontreated, hematoxylin-eosin(HE)-treated and IHC-treated) and a metal plate target and analyzed by MS. Mass spectra were sums of 150 shots acquired in positive ion reflectron TOF mode and the acceleration potential was 18 kV and the lens is set to 3.85 kV. The experimental conditions such as fluence and laser diameter were kept constant for each dilution. Averages of signal intensities for each tag concentration were calculated and graphs of different intensities plotted as function of tag concentrations. From these the detection threshold was determined.

### 1.6 MS analysis

A MALDI TOF/TOF mass spectrometer (Ultraflex II, Bruker Daltonik, Bremen, Germany) was used in this study. It is equipped with a frequency-tripled Nd:YAG laser with a wavelength of 355 nm run at a repetition rate of 200 Hz and a pulse width of about 2 ns. Mass spectra were the sums of 100 shots acquired in positive ion reflectron TOF mode. The acceleration potential was 18 kV and the lens was set to 3.85 kV. The target was moved between 10 and 50 mm from one mass spectrum to the next. The laser focused to roughly 25-30 mm. Flexlmaging (Bruker Daltonik) was used to create MS images. Images were created for m/z 498 6 1, for 532 6 1, and 518 6 1 Th. Flexlmaging requires a visual image to delineate the scanning perimeter. A reference section is used for imaging with immunostaining visualization of the corresponding antigens.

After smoothing of the mass spectra with a Savatzky-Golay filter in FlexAnalysis, peak lists are generated and the peak height at a selected mass linearly translated into an eight bit gray scale of a selected color. Intensity and pixel coordinates are brought together to create false color images. Due to the not complete planarity and varying thickness of the slides and the thickness of the sections the tolerances of masses are larger than usually acceptable for MS. Due to the large number of mass spectra to create an image, the mass spectra were not recalibrated. The m/z were always within 1 m/z of the calculated m/z ratios and tolerances were set at61 m/z.

### 2) Results:

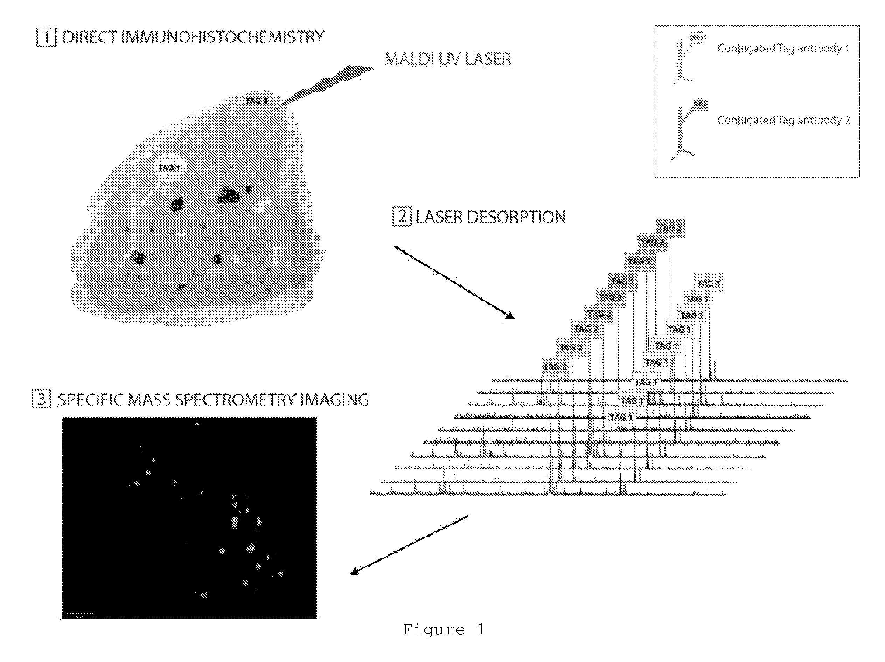

The concept of TAMSIM for the detection of multiple different target proteins in human tissue is illustrated in Figure 1, wherein primary antibodies are conjugated to different mass tags and specific complexes are formed with the antigen in the tissue section. The slide containing the section is mounted on a target plate and introduced into the source of the mass spectrometer. A pulsed UV laser cleaves the tags from their antibodies and releases them into the gas phase. Their m/z values are determined using a TOF analyzer. Acquisition of mass spectra in the scanning mode is used to reconstitute images at specific molecular weight values which each correspond to the localizations of the specific antigens.

The figure 2 shows the Conjugation of a mass tag to an antibody, photocleavage of mass tag conjugated-antibody, and laser desorption. The tagging reagent contains an NHSester as reactive group for covalent attachment to primary amine groups of an antibody. In the mass spectrometer the trityl groups absorb the UV light which results in the cleavage of the C-S bond, creation of a stable carbocation and releases of the tag.

Tagged antibodies (Fig. 2) specifically bind to their target protein in the tissue. The tag is cleaved from the antibody, desorbed with a laser pulse, and sized by the TOF MS. The trityl group of the tag absorbs the impinging UV laser light, which results in the cleavage of the C-S bond leaving a positive charge on the trityl moiety. The positive charge is stabilized by the trityl group. The charge allows the extraction of the cleaved trityl for mass analysis. As the tag acts as its own matrix, it is not necessary to add a matrix to the tissue. For this study three tags, El 307, El 308, and JC14-110 with an m/z of 498, 532, and 518 Th, respectively were used.

As the first improvement of TAMSIM (THIERY et al., Rapid Commun. Mass Spectrom., vol.21, p:823-829, 2007) we demonstrate a strategy of primary antibody labelling. This allowed following conventional protocols closely. Labelling the primary antibody allows increasing the degree of multiplexing as it is not limited by the number of species used for antibody production. NHS esters of the trityl tags are used for the attachment to the primary antibodies. The primary antibodies used here are a rabbit monoclonal to human synaptophysin, a mouse monoclonal to human insulin, a rabbit polyclonal to human chromogranin A, a monoclonal rabbit to human calcitonin, and polyclonal rabbit to human somatostatin. These five antibodies should localize antigens in the Langerhans islets.

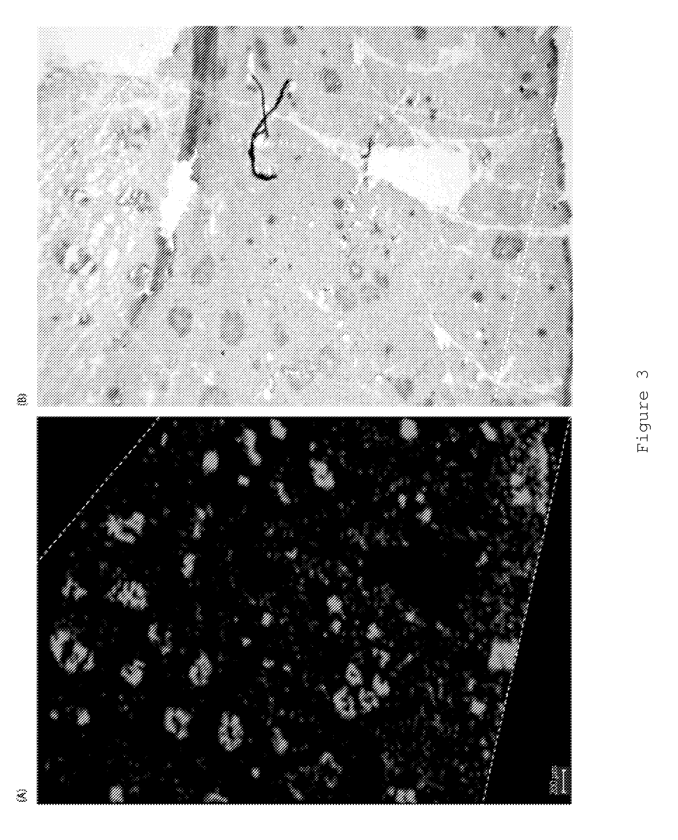

Figure 3 shows the mass spectrometric imaging of synaptophysin which has an m/z of 33800 Th by TAMSIM in a normal human pancreas frozen tissue section. (A) Localization of synaptophysin positive cells by TAMSIM. The monoclonal rabbit antisynaptophysin is conjugated with the tag El 307 which is detected at 498 m/z. The false color green points in the section show the presence of the tag El 307 and thus synaptophysin positive cells. (B) Shows the classical IHC image with the anti-insulin antibody. The dark pink spots corresponds to Langerhans islets and so the synaptophysin-positive cells. The distribution of synaptophysin positive cells in (A) is very similar to that in (B). The section scanned by the mass spectrometer is outlined.

The results established that a slight shift of the mass tag by 1 m/z was observed which is probably due to the thickness of the section or the slide. The green areas in the section correspond to the presence of the El 307 tag and thus synaptophysin-positive cells. IHC was carried out with the classical protocol also using a frozen section. Synaptophysin is spread in discrete spots throughout the section. Comparison of TAMSIM and classical IHC resulted in the same characteristic image and distribution of synaptophysin-positive cells. The TAMSIM image identifies the Langerhans islets well.

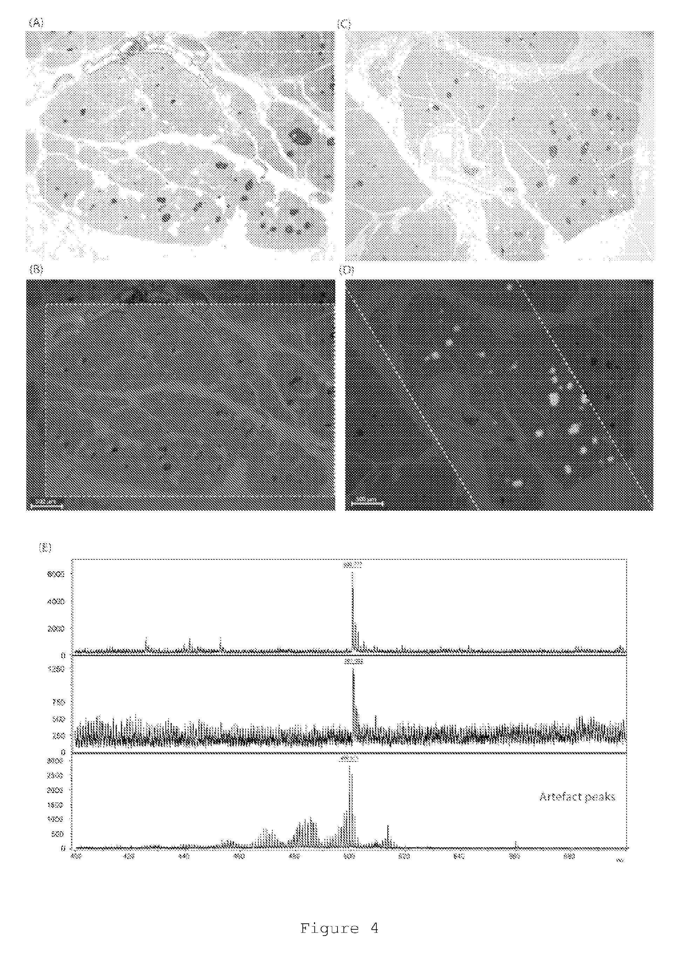

Figure 4 shows in two different sections TAMSIM of chromogranin A and insulin with an m/z of 49 000 and 5805 Th, respectively, in human pancreas embedded paraffin tissue section. (A) Shows the HRP staining with synaptophysin. (B) Shows the MS imaging of the section. The monoclonal mouse anti-human insulin is conjugated with the tag El 307, which has an m/z of 498 Th after cleavage (false color image pink). (C) Shows the HRP staining in another section with synaptophysin. (D) The polyclonal rabbit anti-human chromogranin A is also conjugated with the tag El 307 (green false color image). The image obtained in (A) matches with (B) and the image (C) matches with (D). (E) Shows selected mass spectra from the MS imaging run. The section scanned by the mass spectrometer is outlined.

The results established that the distribution of marker on the Langerhans islets in the mass spectrometric image correlates well with the reference IHC staining of synaptophysin. The example spectra demonstrate a couple of the major drawbacks of using paraffin-embedded sections that are deparaffinized with xylene prior to use. The background of the spectra is high. Unidentified peaks (third example spectrum) are probably due to the paraffin. Obviously these artefact peaks in the spectra also result in an increased background in the images.

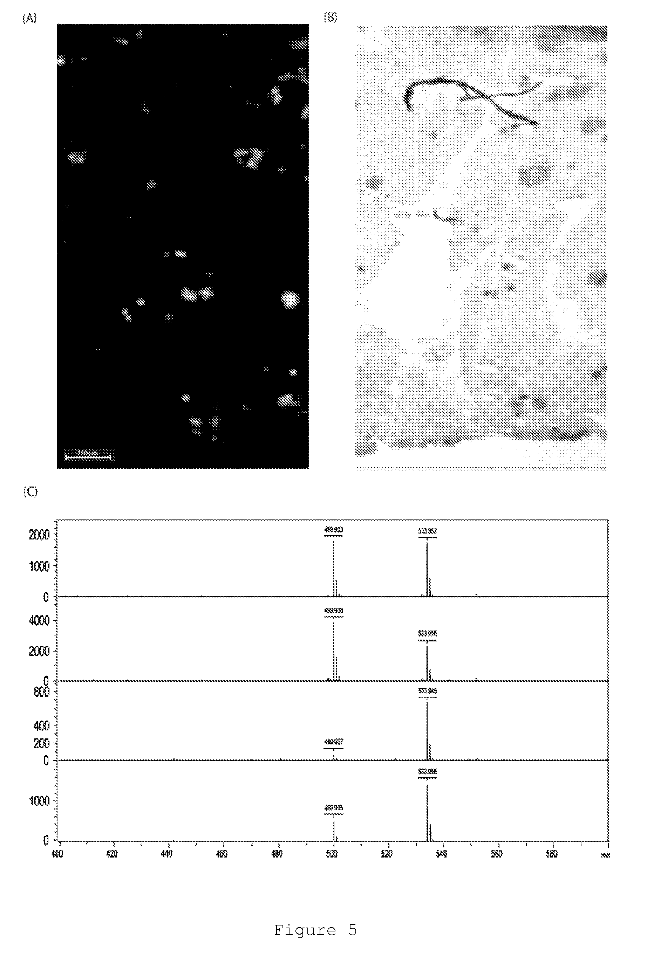

Figure 5 shows TAMSIM of synaptophysin and chromogranin A in a human pancreas frozen tissue sections using two different tags (El 307 with m/z of 499 Th for chromogranin A and El 308 with m/z of 533 Th for synaptophysin) in a single experiment. (A) Shows TAMSIM of chromogranin A and synaptophysin. Chromogranin A is conjugated with the tag El 307, which has an m/z of 498 Th after cleavage (red false color image). Synaptophysin is conjugated with the tag El 308, which has an m/z of 532 Th after cleavage (green false color image). (B) Shows the immunostaining of insulin on the section. (C) Sample mass spectra of tags El 307 and El 308 from different positions on the image. The tags El 307 and El 308 are attached to polyclonal rabbit antihuman chromogranin A and to rabbit monoclonal antisynaptophysin, respectively.

The results show that the distribution of the two markers within the Langerhans islets in the mass spectrometric image correlates well with the reference staining of insulin. In contrast to the experiment shown in Fig. 4 spectra did not have similar artefact peaks and S/N in the individual spectra was better.

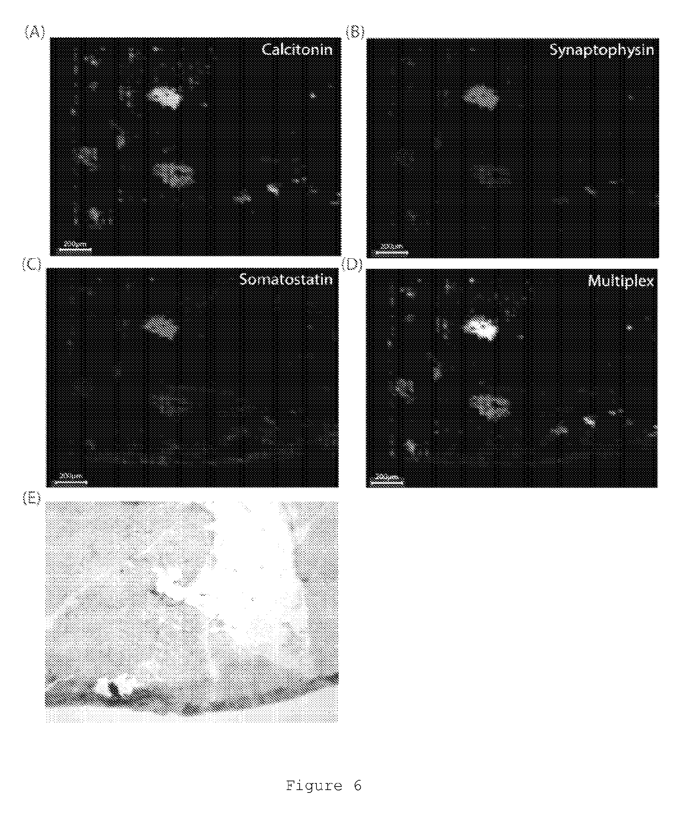

Figure 6 shows a multiplex TAMSIM experiment with three different markers: calcitonin, somatostatin, and synaptophysin in Langerhans islets in a frozen section of human pancreas. (A) Shows TAMSIM of calcitonin. (B) Shows TAMSIM of synaptophysin. (C) Shows TAMSIM of somatostatin. (D) Shows the multiplex of the three biomarkers on the same sample. (E) IHC staining of glucagon.

The results established that synaptophysin stains Langerhans islets more readily than calcitonin and somatostatin. Some background noise is observed for somatostatin. The same effect is observed with IHC and might be due to this specific antibody. The three markers stain the Langerhans islets. Image (E) is the glucagon staining of the islets which should have the same localization as calcitonin and somatostatin. In this instance the IHC staining with glucagon was comparatively faint. The TAMSIM experiment was carried out under the same experimental conditions (on the same tissue section). All three labels were localized in the islets. From this we could conclude that TAMSIM is very sensitive. This led us to investigate the detection threshold of our tags.

A dilution series was deposited on three different tissue sections (not treated, stained with HE, and treated with IHC) and spectra recorded under standardized conditions.

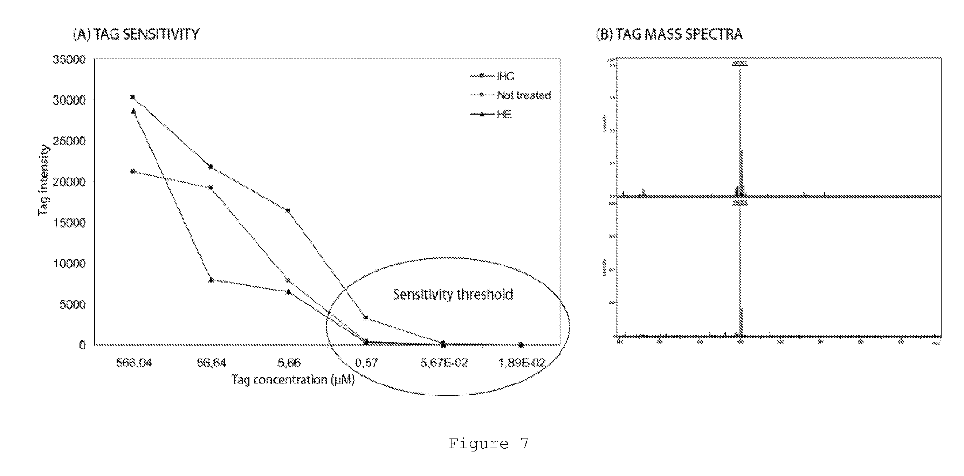

The figure 7 shows the determination of the tag E1 307 sensitivity threshold. (A) Shows the graph of intensity of the tag peak on three different tissue section: not treated, stained with HE and treated with IHC as a function of tag concentration. (B) Shows examples of mass spectra corresponding to two concentrations of the tag.

The detection thresholds were determined to be 5.7 nM with an amount of 28 fmol for the not treated section and the IHC-treated section and 570 nM with an amount of 171 fmol deposited for the HE stained section (Fig. 7). The same samples were also deposited on a steel target and the detection threshold was determined to be 240 pM with an amount of 120 amol deposited. On the steel target the dried spot covered roughly 4 mm2. We do not know the efficiency of our antibody labelling reaction, the number of tags attached per antibody molecule, the number of antigens present per area in the section, and the desorption efficiency of the tag from a section compared to a stainless steel target. However, similar information for IHC with an optical readout is also not available. IHC is largely a qualitative method. As used here TAMSIM is also qualitative. However, it could be envisaged to use it quantitatively if an appropriate standard were included.

## Claims

1. A method of analyzing at least one specific molecule in a sample comprising the steps of:
a) providing a sample;
b) contacting said sample with at least one compound of formula (I) 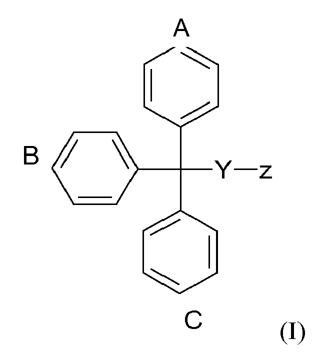 wherein Z binds specifically to said at least one specific molecule, Y is independently a cleavable single bond, linker atom or group, and at least one of the cycles A, B or C is substituted;
c) exposing the sample to a laser beam such that a predetermined laser spot on the sample induces the cleavage of Y to form and released an Ion of formula (II): 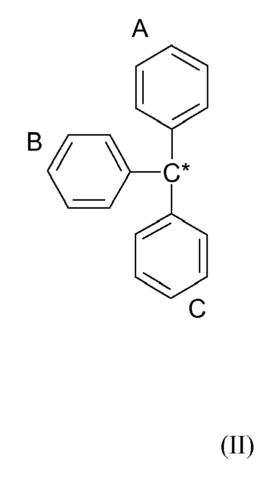 wherein * corresponds to a single positive charge or a single negative charge bearded by the carbon atom , preferably a positive charge;
d) measuring the molecular atomic mass of the released compounds over a range of atomic mass so as to identify and, eventually, quantify the Ion of formula (II);
e) repeating the steps c) to d) to set up an effective scan of the sample; and
f) determining the spatial arrangement and, eventually, the quantity of the at least one specific molecule within the sample.

2. The method of claim 1, wherein said specific molecule is a specific antigen selected in the group consisting of lipids, carbohydrates, peptides and polypeptides.

3. The method of any one of claims 1 or 2, wherein the sample is a tissue section, and preferably a frozen tissue section.

4. The method of any one of claims 1 to 3, wherein said method does not include any step of applying an additional energy absorbent matrix to the sample.

5. The method of claim 2, wherein Z is an antibody or a functional fragment thereof which binds specifically to this specific antigen.

6. The method of any one of claims 1 to 5, wherein said method comprise the step b) of contacting said sample with a compound of formula (I ')

7. The method of any one of claims 1 to 5, wherein 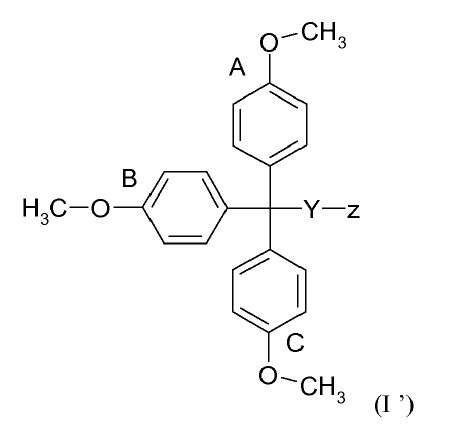 which compound of formula (I '), after exposing of the sample to a laser beam so as to induce the cleavage of Y in step d), form a released Ion of formula (II'):

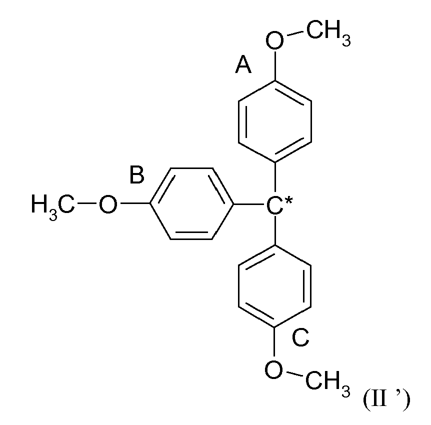

8. The method of any one of claims 1 to 7, wherein said method comprise the step b) of contacting said sample with a compound of formula (I "): 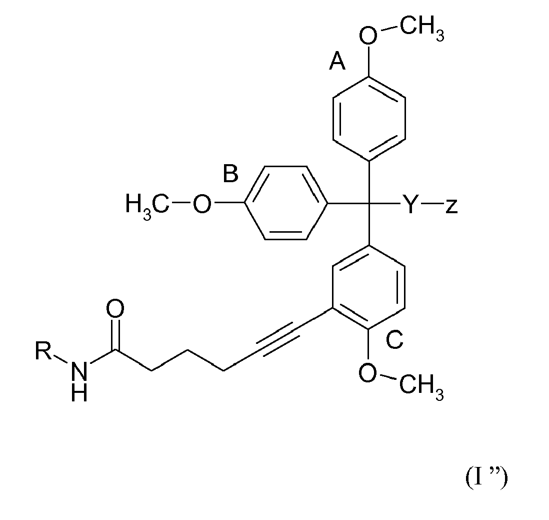 which compound of formula (I "), after exposing of the sample to a laser beam so as to induce the cleavage of Y in step d), form a released Ion of formula (II"): 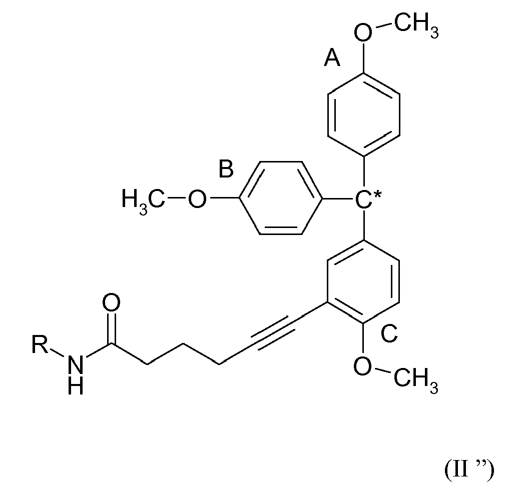 and wherein R is independently a substituent such as H, C₁₋₂₀ hydrocarbyl {e.g. C₁₋₂₀ alkyl, C₁₋₂₀ aryl) or substituted C₁₋₂₀ hydrocarbyl. Preferably, R is H, C₁₋₂₀ alkyl, C₁₋₂₀ aryl, substituted C₁₋₂₀ alkyl or substituted C₁₋₂₀ aryl. More preferably, R is an isopropyl or a phenyl substituted or not with a methyl.

9. The method of any one of claims 1 to 8, wherein the steps c) to e) is carried out with mass spectroscopy in a spectrometer, preferably a MALDI-TOF mass spectrometer.

10. The method of any one of claims 1 to 9, wherein said method is a method of analyzing at least two, three, four or more specific molecules in a sample comprising a step b) of contacting said sample with at least two, three, four or more compounds of formula (I), each compound of formula (I) binding specifically to each specific molecule to be analyzed.

11. A compound of formula (I): 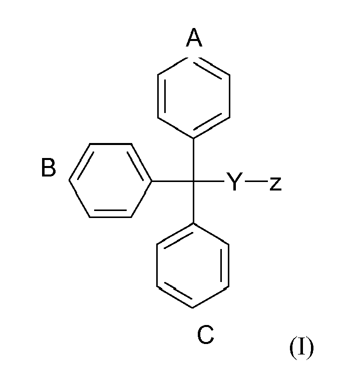 wherein Z binds specifically to the at least one specific molecule as disclosed previously, Y is independently a cleavable single bond, linker atom or group, and at least one of the cycles A, B or C is substituted.

12. The compound of claim 11, wherein said compound has the formula (I '):

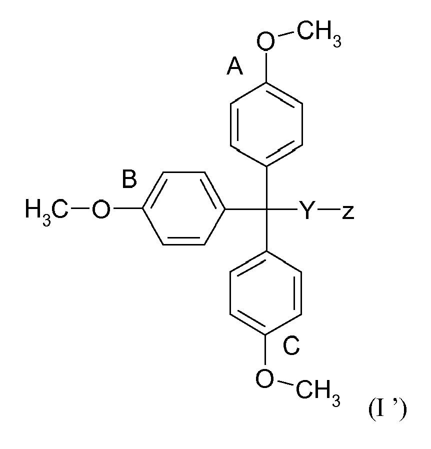

13. The compound of any one of claims 11 or 12, wherein said compound has the formula (I "): 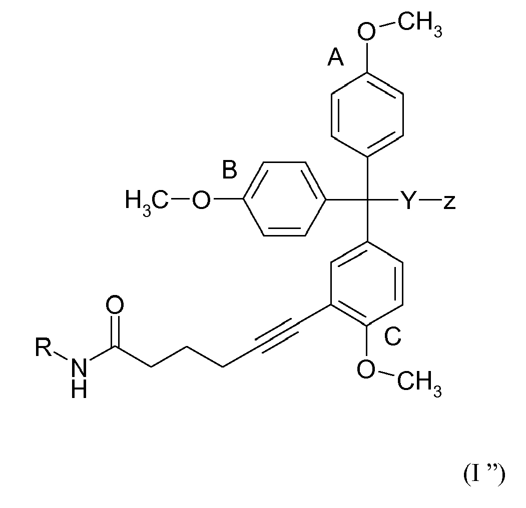 wherein R is independently a substituent such as H, C₁₋₂₀ hydrocarbyl {e.g. C₁₋₂₀ alkyl, C₁₋₂₀ aryl) or substituted C₁₋₂₀ hydrocarbyl. Preferably, R is H, C₁₋₂₀ alkyl, C₁₋₂₀ aryl, substituted C₁₋₂₀ alkyl or substituted C₁₋₂₀ aryl. More preferably, R is an isopropyl or a phenyl substituted or not with a methyl.
